# EUROPEAN PATENT SPECIFICATION

(11) **EP 3 311 687 B1**
(45) Date of publication and mention of the grant of the patent: **19.05.2021**
(21) Application number: 16811605.1
(22) Date of filing: 14.06.2016
(51) Int. Cl.: A44B 18/00, A61F 13/62

(54) **FEMALE MEMBER FOR TOUCH FASTENER**
SCHLAUFENTEIL FÜR KLETTVERSCHLUSS
ÉLÉMENT FEMELLE POUR FERMETURE DE CONTACT

(30) Priority: 19.06.2015 JP 2015123454; 09.06.2016 JP 2016115121
(43) Date of publication of application: 25.04.2018
(73) Proprietor: Nitto Denko Corporation, Ibaraki-shi, Osaka 567-8680 (JP)
(72) Inventor: UCHIDA, Shou, Ibaraki-shi Osaka 567-8680 (JP); IKISHIMA, Shinsuke, Ibaraki-shi Osaka 567-8680 (JP); TAKEDA, Kohei, Ibaraki-shi Osaka 567-8680 (JP); NAKAGAWA, Muneshige, Ibaraki-shi Osaka 567-8680 (JP)
(74) Representative: Müller-Boré & Partner Patentanwälte PartG mbB
(86) International application number: PCT/JP2016/067612
(87) International publication number: WO 2016/204131

(56) References cited:
- EP-A1- 2 596 715
- WO-A1-2008/130807
- JP-A- 2002 315 607
- JP-A- 2012 183 316
- US-A1- 2006 019 572

## Description

### Technical Field

The present invention relates to a hook-and-loop fastener female member. The present invention also relates to a hook-and-loop fastener including the hook-and-loop fastener female member of the present invention. The present invention also relates to a sanitary article including the hook-and-loop fastener female member of the present invention Document WO 2008/130807 A1 discloses a hook-and-loop fastener female member according to the preamble of claim 1.

### Background Art

Various hook-and-loop fasteners are proposed for materials for articles such as sanitary articles, for example, diapers and masks (see, for example, Patent Literatures 1 and 2). When used for, for example, a diaper, a hook-and-loop fastener female member is generally attached to a front surface of a waist portion of the diaper and allowed to engage with a hook-and-loop fastener male member (typically having an engaging hook) fixed to a side surface of the diaper, to thereby complete a hook-and-loop fastener. The hook-and-loop fastener is required to be able to be removed and attached many times.

In recent years, non-woven fabrics have been adopted for many of the engaging layers of hook-and-loop fastener female members (layers on which the engaging hooks of hook-and-loop fastener male members can engage) to be used for sanitary articles (in particular, disposable diapers, supporters, masks, and the like).

However, the related-art hook-and-loop fastener female member including the engaging layer adopting the non-woven fabric does not have a sufficient engaging force with the hook-and-loop fastener male member. In particular, there is a problem in that fluffing of the engaging layer after peeling after engagement with the hook-and-loop fastener male member remarkably lowers the engaging force in reengagement. In addition, the fluffing of the engaging layer causes, for example, another problem in that a baby eats a fluffed portion by accident.

### Citation List

### Patent Literature

[PTL 1] JP 2009-527315 A
[PTL 2] JP 2010-125337 A

### Summary of Invention

### Technical Problem

The present invention has been made to solve the problems of the related art, and an object of the present invention is to provide a hook-and-loop fastener female member including an engaging layer adopting a non-woven fabric in which fluffing of the engaging layer after peeling after engagement with a hook-and-loop fastener male member is effectively suppressed. Another object of the present invention is to provide a hook-and-loop fastener including the hook-and-loop fastener female member of the present invention. Still another object of the present invention is to provide a sanitary article including the hook-and-loop fastener female member of the present invention.

### Solution to Problem

A hook-and-loop fastener female member according to one embodiment of the present invention is a hook-and-loop fastener female member, including:
an engaging layer engageable with a hook-and-loop fastener male member; and
a physical property layer configured to hold the engaging layer,
in which the engaging layer includes a non-woven fabric of fiber,
in which the hook-and-loop fastener female member has an embossed pattern, wherein the embossed pattern is an arc shape, and
in which the hook-and-loop fastener female member has a region free of the embossed pattern in which pieces of the fiber have mutual bonding points

, a distance L between two adjacent embossments in a plurality of embossments forming the embossed pattern on any line in an MD direction is 10 mm or less, the diameter of the fiber of the non-woven fabric in the engaging layer is from 5 µm to 60 µm, and the ratio of the area of the welded portion formed by the embossed pattern to the area of the entire surface of the hook-and-loop fastener female member of from 12% to 23%.

In one embodiment, the distance L between the two adjacent embossments in the plurality of embossments forming the embossed pattern on any line in the MD direction is 7 mm or less.

In one embodiment, a distance L between two adjacent embossments in a plurality of embossments forming the embossed pattern on any line in an MD direction is 1 mm or more.

In one embodiment, the distance L between the two adjacent embossments in the plurality of embossments forming the embossed pattern on any line in the MD direction is 3 mm or more.

In one embodiment, an embossment width W of each of a plurality of embossments forming the embossed pattern is 0.1 mm or more.

In one embodiment, the embossment width W of each of the plurality of embossments forming the embossed pattern is 0.5 mm or more.

In one embodiment, an embossment width W of each of a plurality of embossments forming the embossed pattern is 3.0 mm or less.

In one embodiment, the embossment width W of each of the plurality of embossments forming the embossed pattern is 1.5 mm or less.

In one embodiment, a ratio L/W of a distance L between two adjacent embossments in a plurality of embossments forming the embossed pattern to an embossment width W of each of the plurality of embossments forming the embossed pattern is from 0.3 to 100.

In one embodiment, the ratio L/W of the distance L between the two adjacent embossments in the plurality of embossments forming the embossed pattern to the embossment width W of each of the plurality of embossments forming the embossed pattern is from 2 to 10.

In one embodiment, a surface of the fiber and a surface of the physical property layer on an engaging layer side contain the same kind of polymer.

In one embodiment, the polymer includes polyolefin.

In one embodiment, the non-woven fabric includes a thermal point-bonded non-woven fabric.

In one embodiment, the thermal point-bonded non-woven fabric includes a spunbonded non-woven fabric.

In one embodiment, a total basis weight, which is a sum of a basis weight of the non-woven fabric in the engaging layer and a basis weight of a non-woven fabric in the physical property layer, is 60 g/m² or less.

In one embodiment, the total basis weight, which is the sum of the basis weight of the non-woven fabric in the engaging layer and the basis weight of the non-woven fabric in the physical property layer, is 47 g/m² or less.

In one embodiment, a total basis weight, which is a sum of a basis weight of the non-woven fabric in the engaging layer and a basis weight of a non-woven fabric in the physical property layer, is 30 g/m² or more.

A hook-and-loop fastener according to one embodiment of the present invention includes: the hook-and-loop fastener female member according to the embodiment of the present invention; and a hook-and-loop fastener male member configured to engage with the hook-and-loop fastener female member.

A sanitary article according to one embodiment of the present invention includes the hook-and-loop fastener female member according to the embodiment of the present invention.

### Advantageous Effects of Invention

According to the present invention, the hook-and-loop fastener female member including an engaging layer adopting a non-woven fabric in which fluffing of the engaging layer after peel ing after engagement with a hook-and-loop fastener male member is effectively suppressed can be provided. The hook-and-loop fastener including such hook-and-loop fastener female member can also be provided. The sanitary article including such hook-and-loop fastener female member can also be provided.

### Brief Description of Drawings

FIG. **1** is a schematic plan view of a hook-and-loop fastener female member according to a preferred embodiment of the present invention.
FIG. **2** is a schematic plan view of a hook-and-loop fastener female member according to another preferred embodiment of the present invention.
FIG. **3** is a schematic plan view of a hook-and-loop fastener female member according to still another preferred embodiment of the present invention.

### Description of Embodiments

### <<Hook-and-loop Fastener Female Member>>

Ahook-and-loop fastener female member of the present invention is a hook-and-loop fastener female member including an engaging layer engageable with a male member (sometimes referred to as "mechanical hook member") . The engaging layer of the hook-and-loop fastener female member is specifically a layer on which an engaging hook (or something having properties equivalent to those of the engaging hook) of a hook-and-loop fastener male member is engageable. A product including the hook-and-loop fastener female member of the present invention and a hook-and-loop fastener male member configured to engage with the hook-and-loop fastener female member serves as a hook-and-loop fastener.

The hook-and-loop fastener female member of the present invention includes the engaging layer engageable with a hook-and-loop fastener male member and a physical property layer configured to hold the engaging layer. The hook-and-loop fastener female member of the present invention may include any appropriate other member as long as the hook-and-loop fastener female member includes such engaging layer and physical property layer and the effect of the present invention is not impaired. The hook-and-loop fastener female member of the present invention is preferably formed of the engaging layer engageable with a hook-and-loop fastener male member and the physical property layer conf igured to hold the engaging layer.

The thickness of the hook-and-loop fastener female member of the present invention may be set to any appropriate thickness depending on the purpose. Typically, the thickness of the hook-and-loop fastener female member of the present invention is preferably from 0.2 mm to 5.0 mm, more preferably from 0.3 mm to 4.0 mm, still more preferably from 0.5 mm to 3.0 mm, particularly preferably from 0.5 mm to 2.0 mm. In the present invention, the thickness of the hook-and-loop fastener female member (non-woven fabric) is measured on the basis of a method to be described later.

The engaging layer includes a non-woven fabric of fiber. The number of layers of the engaging layer may be only one, or may be two or more. The engaging layer is preferably formed only of the non-woven fabric of fiber.

The number of kinds of the non-woven fabric of fiber included in the engaging layer may be only one, or may be two or more.

Examples of the non-woven fabric of fiber included in the engaging layer include a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (e.g. , TYVEK™ from DuPont), and a carded non-woven fabric. In this connection, the spunbonded non-woven fabric may be said to be a thermal point-bonded non-woven fabric when viewed from the aspect of having its fiber thermally welded. On the other hand, the thermally bonded non-woven fabric is a thermal point-bonded carded non-woven fabric. Therefore, the spunbonded non-woven fabric and the thermally bonded non-woven fabric herein refer to clearly different things. Such thermal point-bonded non-woven fabric is given as the non-woven fabric of fiber included in the engaging layer in one preferred embodiment. In addition, of the above-mentioned non-woven fabrics, a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, or a spunbonded meltblown meltblown spunbonded non-woven fabric is preferred, a spunbonded non-woven fabric, a thermally bonded non-woven fabric, or an air-through non-woven fabric is more preferred, a spunbonded non-woven fabric or an air-through non-woven fabric is still more preferred, and a spunbonded non-woven fabric is particularly preferred. When, for example, a spunbonded non-woven fabric (thermal point-bonded spunbonded non-woven fabric), a thermally bonded non-woven fabric (thermal point-bonded carded non-woven fabric), or an air-through non-woven fabric (hot air-bonded airlaid non-woven fabric) is used as the non-woven fabric of fiber included in the engaging layer, pieces of the fiber forming the non-woven fabric included in the engaging layer can have mutual bonding points. With this, when the hook-and-loop fastener female member of the present invention has an embossed pattern, not only pieces of the fiber forming the non-woven fabric included in the engaging layer have firm mutual bonding points in the embossed pattern portions as a result of embossing treatment, but also pieces of the fiber forming the non-woven fabric included in the engaging layer have mutual bonding points in a region free of the embossed pattern. When such structure can be achieved, the hook-and-loop fastener female member of the present invention is a hook-and-loop fastener female member including an engaging layer adopting a non-woven fabric in which fluffing of the engaging layer after peeling after engagement with a hook-and-loop fastener male member can be effectively suppressed. Meanwhile, for example, when a spunlace non-woven fabric, for example, is used as the non-woven fabric of fiber included in the engaging layer, pieces of the fiber forming the non-woven fabric included in the engaging layer hardly have mutual bonding points. Accordingly, when the hook-and-loop fastener female member of the present invention has an embossed pattern, pieces of the fiber forming the non-woven fabric included in the engaging layer have firm mutual bonding points in the embossed pattern portions as a result of embossing treatment, but pieces of the fiber forming the non-woven fabric included in the engaging layer hardly have mutual bonding points in the region free of the embossed pattern. Accordingly, there is a fear that the effect of the present invention cannot be expressed.

Herein, that pieces of the fiber forming the non-woven fabric of fiber included in the engaging layer have mutual bonding points typically means that mutual fusing points of pieces of the fiber can be recognized in observation of the non-woven fabric of fiber included in the engaging layer with a microscope or the like.

In the case where the non-woven fabric of fiber included in the engaging layer is a spunbonded non-woven fabric, the number of bonding points per unit area to be recognized in the region free of the embossed pattern in observation of the non-woven fabric of fiber included in the engaging layer with an optical microscope in a 17 mm×13 mm field of view (at a magnification of 7.5) is preferably from 10 to 200, more preferably from 30 to 150, still more preferably from 50 to 100. In the case where the non-woven fabric of fiber included in the engaging layer is a spunbonded non-woven fabric, when the number of bonding points per unit area to be recognized in observation of the non-woven fabric of fiber included in the engaging layer with an optical microscope falls within the above-mentioned range, fluffing of the engaging layer after peeling after engagement with a hook-and-loop fastener male member can be more effectively suppressed in the hook-and-loop fastener female member of the present invention.

In the case where the non-woven fabric of fiber included in the engaging layer is an air-through non-woven fabric, the number of bonding points per unit area to be recognized in the region free of the embossed pattern in observation of the non-woven fabric of fiber included in the engaging layer with an SEM in a 1.3 mm×1.0 mm field of view (at a magnification of 100) is preferably 1 or more, more preferably from 2 to 100, still more preferably from 5 to 50. In the case where the non-woven fabric of fiber included in the engaging layer is an air-through non-woven fabric, when the number of bonding points per unit area to be recognized in observation of the non-woven fabric of fiber included in the engaging layer with an SEM falls within the above-mentioned range, fluffing of the engaging layer after peeling after engagement with a hook-and-loop fastener male member can be more effectively suppressed in the hook-and-loop fastener female member of the present invention.

In the case where the non-woven fabric of fiber included in the engaging layer is a thermally bonded non-woven fabric, the number of bonding points per unit area to be recognized in the region free of the embossed pattern in observation of the non-woven fabric of fiber included in the engaging layer with an optical microscope in a 17 mm×13 mm field of view (at a magnification of 7. 5) is preferably from 10 to 200, more preferably from 30 to 150, still more preferably from 50 to 100. In the case where the non-woven fabric of fiber included in the engaging layer is a thermally bonded non-woven fabric, when the number of bonding points per unit area to be recognized in observation of the non-woven fabric of fiber included in the engaging layer with an optical microscope falls within the above-mentioned range, fluffing of the engaging layer after peeling after engagement with a hook-and-loop fastener male member can be more effectively suppressed in the hook-and-loop fastener female member of the present invention.

The non-woven fabric of fiber included in the engaging layer may contain fiber that is a homogeneous structural body, or may contain a composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992) .

Any appropriate fiber may be adopted as the fiber forming the non-woven fabric included in the engaging layer as long as the effect of the present invention is not impaired. For example, such fiber contains polyolefin (such as polypropylene or polyethylene), polyester, polyamide, polyurethane, an elastomer, rayon, cellulose, acrylic , a copolymer thereof, or a blend thereof, or a mixture thereof . Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and α-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

The fiber forming the non-woven fabric included in the engaging layer may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

The fiber forming the non-woven fabric included in the engaging layer may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

In the hook-and-loop fastener female member of the present invention, the density of the non-woven fabric in the engaging layer is preferably from 5 kg/m³ to 100 kg/m³, more preferably from 10 kg/m³ to 100 kg/m³, still more preferably from 10 kg/m³ to 80 kg/m³, still more preferably from 10 kg/m³ to 70 kg/m³, particularly preferably from 10 kg/m³ to 60 kg/m³, most preferably from 20 kg/m³ to 50 kg/m³, because the effect of the present invention can be more effectively expressed. In the hook-and-loop fastener female member of the present invention, when the density of the non-woven fabric in the engaging layer falls within the above-mentioned range, both high shearing stress and high peeling strength can be achieved as the engaging force with a hook-and-loop fastener male member, and hence, in a disposable diaper or the like, the problem of slippage, for example, at the time of wearing or after excretion can be effectively eliminated. In the hook-and-loop fastener female member of the present invention, when the density of the non-woven fabric in the engaging layer is lower than 5 kg/m³, there is a fear that a hook-and-loop fastener male member may be hardly hooked or productivity may be poor, leading to an increased cost. When the density of the non-woven fabric in the engaging layer is higher than 100 kg/m³, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed. In the present invention, the density (kg/m³) of the non-woven fabric in the engaging layer is a value calculated from the basis weight (X g/m²) of the non-woven fabric and the thickness (Y mm) of the non-woven fabric measured on the basis of a method to be described later. More specifically, the density (kg/m³) of the non-woven fabric in the engaging layer is calculated as X/Y (kg/m³).

In the hook-and-loop fastener female member of the present invention, the diameter of the fiber (hereinafter sometimes referred to simply as "fiber diameter") of the non-woven fabric in the engaging layer is from 5 µm to 60 µm, preferably from 10 µm to 60 µm, more preferably from 10 µm to 50 µm, still more preferably from 10 µm to 40 µm, particularly preferably from 15 µm to 40 µm, most preferably from 20 µm to 40 µm, because the effect of the present invention can be more effectively expressed. In the hook-and-loop fastener female member of the present invention, when the diameter of the fiber of the non-woven fabric in the engaging layer falls within the above-mentioned range, both high shearing stress and high peeling strength can be achieved as the engaging force with a hook-and-loop fastener male member, and hence, in a disposable diaper or the like, the problem of slippage, for example, at the time of wearing or after excretion can be effectively eliminated. In the hook-and-loop fastener female member of the present invention, when the diameter of the fiber of the non-woven fabric in the engaging layer is smaller than 5 µm, there is a fear that as the engaging force with a hook-and-loop fastener male member, particularly the shearing stress may lower. When the diameter of the fiber of the non-woven fabric in the engaging layer is larger than 60 µm, there is a fear that engagement with a hook-and-loop fastener male member may become difficult or production speed may lower, leading to an increased cost. In the present invention, the diameter of the fiber of the non-woven fabric in the engaging layer (fiber diameter) is measured on the basis of a method to be described later.

In the hook-and-loop fastener female member of the present invention, the basis weight of the non-woven fabric in the engaging layer is preferably from 10 g/m² to 60 g/m², more preferably from 12 g/m² to 50 g/m², still more preferably from 15 g/m² to 40 g/m², particularly preferably from 15 g/m² to 30 g/m², most preferably from 15 g/m² to 25 g/m². In the hook-and-loop fastener female member of the present invention, when the basis weight of the non-woven fabric in the engaging layer falls within the above-mentioned range, there can be provided a hook-and-loop fastener female member including an engaging layer adopting a non-woven fabric in which fluffing of the engaging layer after peeling after engagement with a hook-and-loop fastener male member is more effectively suppressed.

Any appropriate material may be adopted as a material for the physical property layer as long as the effect of the present invention is not impaired. Examples of the material for the physical property layer include a non-woven fabric of fiber and a film, which are preferred because the effect of the present invention can be more expressed. Of those, a non-woven fabric of fiber is more preferred because the effect of the present invention can be more effectively expressed. That is, the hook-and-loop fastener female member of the present invention is preferably formed only of non-woven fabrics because the effect of the present invention can be more effectively expressed.

When the material for the physical property layer is a non-woven fabric of fiber, the number of kinds of the non-woven fabric may be only one, or may be two or more.

When the material for the physical property layer is a non-woven fabric of fiber, examples of the non-woven fabric include a spunbonded non-woven fabric, a thermally bonded non-woven fabric, a bonded and joined non-woven fabric, an air-through non-woven fabric, a meltblown non-woven fabric, a spunlace non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (such as TYVEK™ from DuPont), and a carded non-woven fabric.

When the material for the physical property layer is a non-woven fabric of fiber, the non-woven fabric may contain fiber that is a homogeneous structural body, or may contain a composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler, " E.A. Vaughn, Association of the Nonwoven Fabrics Industry, third edition (1992).

When the material for the physical property layer is a non-woven fabric of fiber, any appropriate fiber may be adopted as the fiber as long as the effect of the present invention is not impaired. For example, such fiber contains polyolefin (such as polypropylene or polyethylene), polyester, polyamide, polyurethane, an elastomer, rayon, cellulose, acrylic, a copolymer thereof, or a blend thereof, or a mixture thereof. Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and α-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

When the material for the physical property layer is a non-woven fabric of fiber, the fiber forming the non-woven fabric may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

When the material for the physical property layer is a non-woven fabric of fiber, the fiber forming the non-woven fabric may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

When the material for the physical property layer is a film, any appropriate material may be adopted as a material for the film as long as the effect of the present invention is not impaired. Examples of such material include an unstretched polypropylene film, a stretched polypropylene film, and a polyethylene film each having a thickness of from 10 µm to 60 µm, which are preferred because the effect of the present invention can be more effectively expressed.

When the physical property layer is a non-woven fabric of fiber, the non-woven fabric in the physical property layer has a basis weight of preferably from 10 g/m² to 40 g/m², more preferably from 10 g/m² to 30 g/m², still more preferably from 10 g/m² to 25 g/m², particularly preferably from 10 g/m² to 20 g/m². In the case where the physical property layer is a non-woven fabric of fiber, when the basis weight of the non-woven fabric in the physical property layer falls within the above-mentioned range, shrinkage deformation in a width direction hardly occurs during web handling, cost competitiveness is excellent, printability is satisfactory, the see-through property of printing is satisfactory, pressure-sensitive adhesive application is easy, the applied pressure-sensitive adhesive hardly exudes to the engaging surface, and flexibility is satisfactory.

When the physical property layer is a non-woven fabric of fiber, the diameter of the fiber is preferably 40 µm or less, more preferably from 1 µm to 40 µm, still more preferably from 1 µm to 30 µm, particularly preferably from 1 µm to 25 µm, most preferably from 1 µm to 20 µm. In the case where the physical property layer is a non-woven fabric of fiber, when the diameter of the fiber falls within the above-mentioned range, shrinkage deformation in a width direction hardly occurs during web handling, cost competitiveness is excellent, printability is satisfactory, the see-through property of printing is satisfactory, pressure-sensitive adhesive application is easy, the applied pressure-sensitive adhesive hardly exudes to the engaging surface, and flexibility is satisfactory. In the case where the physical property layer is a non-woven fabric of fiber, when the diameter of the fiber is larger than 40 µm, there is a fear that shrinkage deformation in a width direction may be liable to occur during web handling, cost competitiveness may be inferior, printability may be poor, pressure-sensitive adhesive application may be difficult, and the applied pressure-sensitive adhesive may have a risk of exuding to the engaging surface.

When the physical property layer is a non-woven fabric of fiber, in the hook-and-loop fastener female member of the present invention, the density of the non-woven fabric is preferably from 5 kg/m³ to 200 kg/m³, more preferably from 20 kg/m³ to 150 kg/m³, still more preferably from 50 kg/m³ to 150 kg/m³, still more preferably from 50 kg/m³ to 120 kg/m³, particularly preferably from 60 kg/m³ to 120 kg/m³, most preferably from 70 kg/m³ to 120 kg/m³, because the effect of the present invention can be more effectively expressed. In the present invention, the density (kg/m³) of the non-woven fabric in the physical property layer is a value calculated from the basis weight (X g/m²) of the non-woven fabric and the thickness (Y mm) of the non-woven fabric measured on the basis of a method to be described later. More specifically, the density (kg/m³) of the non-woven fabric in the physical property layer is calculated as X/Y (kg/m³).

When the physical property layer is a non-woven fabric of fiber, a laminate of different kinds of non-woven fabrics of fiber (e.g., a laminate of spunbonded non-woven fabric/meltblown non-woven fabric/spunbonded non-woven fabric) may be adopted.

When the diameter of the fiber significantly varied in a thickness direction (e. g. , SMS or SSMMS), the same number of diameters of the fiber were measured for N=5 or more in each piece of the fiber, and the average value of the measured diameters was defined as the diameter of the fiber. A portion having a locally small thickness due to heat fusion or the like as in spunbond, spunmelt, or the like was not included.

When the physical property layer is a film, its thickness is preferably 60 µm or less, more preferably from 10 µm to 50 µm, still more preferably from 10 µm to 40 µm, particularly preferably from 10 µm to 30 µm, most preferably from 15 µm to 25 µm. In the case where the physical property layer is a film, when its thickness falls within the above-mentioned range, shrinkage deformation in a width direction hardly occurs during web handling, cost competitiveness is excellent, the see-through property of printing is satisfactory, pressure-sensitive adhesive application is easy, and flexibility is satisfactory. In the physical property layer is a film, when its thickness is larger than 60 µm, there is a fear that cost competitiveness may be inferior, the see-through property of printing may degrade, and flexibility may degrade.

In the hook-and-loop fastener female member of the present invention, the total basis weight, which is the sum of the basis weight of the non-woven fabric in the engaging layer and the basis weight of the non-woven fabric in the physical property layer, is preferably 60 g/m² or less, more preferably from 10 g/m² to 57 g/m², still more preferably from 15 g/m² to 53 g/m², particularly preferably from 20 g/m² to 50 g/m², most preferably from 30 g/m² to 47 g/m². In the hook-and-loop fastener female member of the present invention, when the total basis weight, which is the sum of the basis weight of the non-woven fabric in the engaging layer and the basis weight of the non-woven fabric in the physical property layer, falls within the above-mentioned range, shrinkage deformation in a width direction is still less liable to occur during web handling, cost competitiveness is more excellent, printability is more satisfactory, the see-through property of printing is more satisfactory, pressure-sensitive adhesive application is easier, the applied pressure-sensitive adhesive is still less liable to exude to the engaging surface, and flexibility is more satisfactory.

In the hook-and-loop fastener female member of the present invention, it is preferred that the surface of the fiber forming the non-woven fabric included in the engaging layer and the surface of the physical property layer on an engaging layer side contain the same kind of polymer. When the surface of the fiber forming the non-woven fabric included in the engaging layer and the surface of the physical property layer on the engaging layer side contain the same kind of polymer, there can be provided a hook-and-loop fastener female member in which fluffing of the engaging layer after peeling after engagement with a hook-and-loop fastener male member is more effectively suppressed. In this case, the "surface of the fiber forming the non-woven fabric included in the engaging layer" may be any surface of the fiber, and encompasses, for example, a sheath portion in fiber having a core-sheath structure.

When the surface of the fiber forming the non-woven fabric included in the engaging layer and the surface of the physical property layer on the engaging layer side contain the same kind of polymer, any appropriate polymer may be adopted as the polymer as long as the effect of the present invention is not impaired. Such polymer is preferably polyolefin. When the surface of the fiber forming the non-woven fabric included in the engaging layer and the surface of the physical property layer on the engaging layer side contain the same kind of polyolefin, there can be provided a hook-and-loop fastener female member in which fluffing of the engaging layer after peeling after engagement with a hook-and-loop fastener male member is even more effectively suppressed.

The hook-and-loop fastener female member of the present invention has an embossed pattern. Such embossed pattern is preferably formed by embossing treatment. Specific examples of the embossed pattern include a continuous grid shape, a discontinuous grid shape, a continuous curve shape, a discontinuous curve shape, a continuous zigzag shape, a discontinuous zigzag shape, a continuous linear shape, a discontinuous linear shape, a circle shape, an ellipse shape, a hollow circle shape, a hollow ellipse shape, an arc shape, and a hollow arc shape.

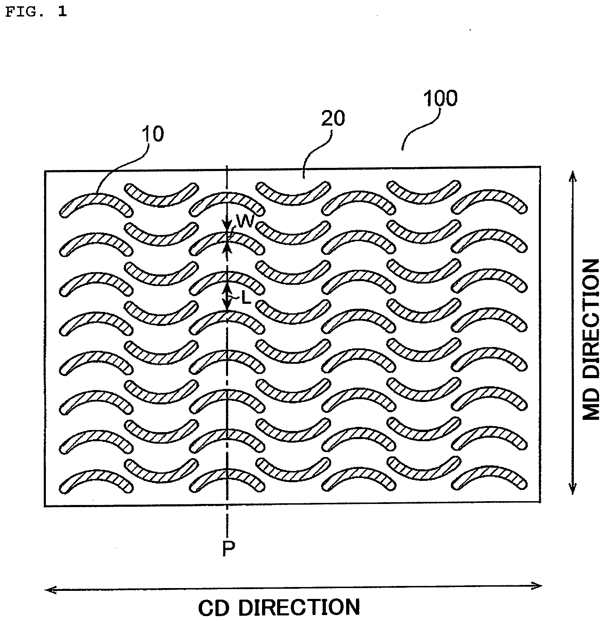

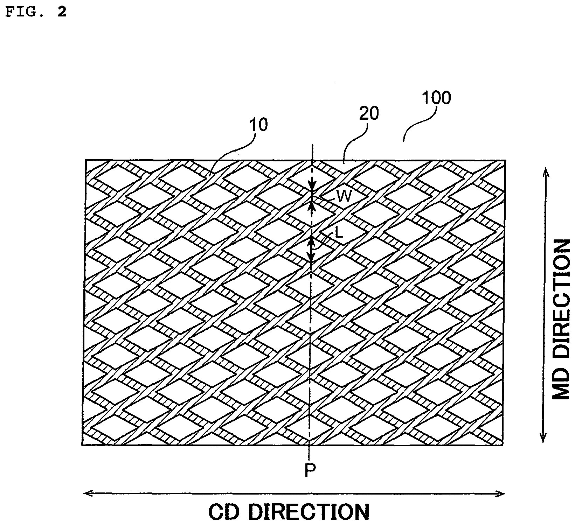

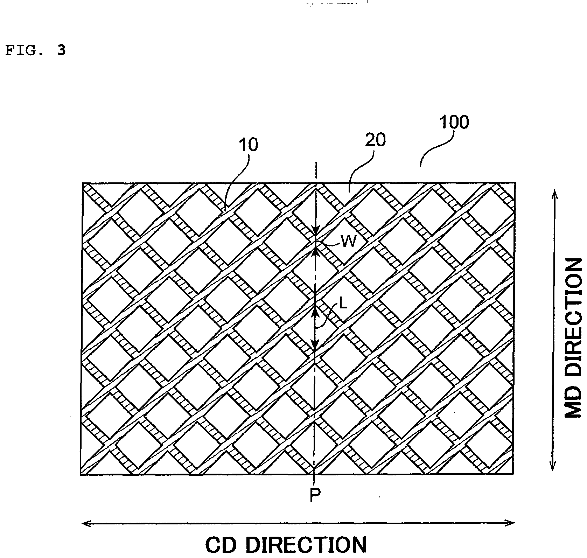

The embossed pattern of the hook-and-loop fastener female member of the present invention is preferably a discontinuous embossed pattern, more preferably an embossed pattern of an arc shape, a rhombus shape, or a grid shape, still more preferably an embossed pattern of an arc shape, because the effect of the present invention can be more effectively expressed. A schematic plan view of the hook-and-loop fastener female member of the present invention having the embossed pattern of an arc shape is illustrated in FIG. **1****.** A schematic plan view of the hook-and-loop fastener female member of the present invention having the embossed pattern of a rhombus shape is illustrated in FIG. **2****.** A schematic plan view of the hook-and-loop fastener female member of the present invention having the embossed pattern of a grid shape is illustrated in FIG. **3****.**

In FIG. **1****,** a hook-and-loop fastener female member **100** of the present invention has a plurality of embossments **10** forming an embossed pattern of an arc shape. In FIG. 1, the hook-and-loop fastener female member of the present invention has a region **20** free of the embossed pattern.

In FIG. **2****,** a hook-and-loop fastener female member **100** of the present invention has an embossment **10** forming an embossed pattern of a rhombus shape. In FIG. **2****,** the hook-and-loop fastener female member of the present invention has a region **20** free of the embossed pattern.

In FIG. **3****,** a hook-and-loop fastener female member **100** of the present invention has an embossment **10** forming an embossed pattern of a grid shape. In FIG. **3****,** the hook-and-loop fastener female member of the present invention has a region **20** free of the embossed pattern.

In the hook-and-loop fastener female member of the present invention, when, for example, a spunbonded non-woven fabric or an air-through non-woven fabric is used as the non-woven fabric of fiber included in the engaging layer, pieces of the fiber forming the non-woven fabric included in the engaging layer can have mutual bonding points. With this, when the hook-and-loop fastener female member of the present invention has an embossed pattern, not only pieces of the fiber forming the non-woven fabric included in the engaging layer have firm mutual bonding points in the embossed pattern portions (e.g., the portions of the plurality of embossments **10** in FIG. **1****)** as a result of embossing treatment, but also pieces of the fiber forming the non-woven fabric included in the engaging layer have mutual bonding points in the region free of the embossed pattern (e.g., the region **20** free of the embossed pattern in FIG. **1****).** When such structure can be achieved, the hook-and-loop fastener female member of the present invention is a hook-and-loop fastener female member including an engaging layer adopting a non-woven fabric in which fluffing of the engaging layer after peeling after engagement with a hook-and-loop fastener male member can be effectively suppressed. Meanwhile, for example, when a spunlace non-woven fabric, for example, is used as the non-woven fabric of fiber included in the engaging layer, pieces of the fiber forming the non-woven fabric included in the engaging layer hardly have mutual bonding points. Accordingly, when the hook-and-loop fastener female member of the present invention has an embossed pattern, pieces of the fiber forming the non-woven fabric included in the engaging layer have firm mutual bonding points in the embossed pattern portions (e.g., the portions of the plurality of embossments **10** in FIG. **1****)** as a result of embossing treatment, but pieces of the fiber forming the non-woven fabric included in the engaging layer hardly have mutual bonding points in the region free of the embossed pattern (e.g., the region **20** free of the embossed pattern in FIG. **1**). Accordingly, there is a fear that the effect of the present invention cannot be expressed.

In the hook-and-loop fastener female member of the present invention, the embossment width of each of the embossments forming the embossed pattern is preferably from 0.1 mm to 3.0 mm, more preferably from 0.3 mm to 2.0 mm, still more preferably from 0.3 mm to 1.5 mm, particularly preferably from 0.5 mm to 1.5 mm, most preferably from 0.5 mm to 1.2 mm. When the embossment width falls within the above-mentioned range, there can be provided a hook-and-loop fastener female member including an engaging layer adopting a non-woven fabric in which fluffing of the engaging layer after peeling after engagement with a hook-and-loop fastener male member is more effectively suppressed. The embossment width in the hook-and-loop fastener female member of the present invention refers to, for example, a width W of each of the embossments **10** in an MD direction as illustrated in each of FIG. **1** to FIG. **3****.**

In the hook-and-loop fastener female member of the present invention, the distance between two adjacent embossments in the embossments forming the embossed pattern on any line in the MD direction is preferably 10 mm or less, more preferably from 1 mm to 10 mm, still more preferably from 1.5 mm to 9 mm, particularly preferably from 2 mm to 8 mm, most preferably from 2.5 mm to 7 mm. When the distance between two adjacent embossments in the embossments forming the embossed pattern on any line in the MD direction falls within the above-mentioned range, there can be provided a hook-and-loop fastener female member including an engaging layer adopting a non-woven fabric in which fluffing of the engaging layer after peeling after engagement with a hook-and-loop fastener male member is more effectively suppressed.

The distance between two adjacent embossments in the embossments forming the embossed pattern on a line in the MD direction in the hook-and-loop fastener female member of the present invention is, in the case of the embossed pattern of an arc shape as illustrated in FIG. **1****,** for example, a distance L between two adjacent embossments on a line P in the MD direction illustrated in FIG. **1** (which may be a line in the MD direction at any position in a CD direction), and in the case of the embossed pattern of a rhombus shape as illustrated in FIG. **2** or the embossed pattern of a grid shape as illustrated in FIG. **3****,** for example, the distance L between two adjacent embossments (intersections of the embossments) on the line P in the MD direction illustrated in FIG. **2** or FIG. **3** (line in the MD direction at a position in the CD direction passing through intersections of the embossments) . That is, the distance L is the maximum value of the distance between two adjacent embossments (sometimes referred to as "maximum embossment-to-embossment distance") on a line in the MD direction in the embossed pattern (which may be a line in the MD direction at any position in the CD direction).

In the hook-and-loop fastener female member of the present invention, a ratio L/W of the distance L between two adjacent embossments in the embossments forming the embossed pattern to the embossment width W of each of the embossments forming the embossed pattern is preferably from 0.3 to 100, more preferably from 1 to 50, still more preferably from 2 to 10. When the ratio L/W of the distance L between two adjacent embossments in the embossments forming the embossed pattern to the embossment width W of each of the embossments forming the embossed pattern falls within the above-mentioned range, the hook-and-loop fastener female member of the present invention is a hook-and-loop fastener female member including an engaging layer adopting a non-woven fabric in which fluffing of the engaging layer after peeling after engagement with a hook-and-loop fastener male member can be more effectively suppressed.

The depth of each of the embossments is preferably from 0.1 mm to 2.0 mm, more preferably from 0.2 mm to 1.8 mm, still more preferably from 0.3 mm to 1.5 mm, particularly preferably from 0.5 mm to 1.5 mm, most preferably from 0.7 mm to 1.2 mm. When the depth of each of the embossments falls within the above-mentioned range, there can be provided a hook-and-loop fastener female member including an engaging layer adopting a non-woven fabric in which fluffing of the engaging layer after peeling after engagement with a hook-and-loop fastener male member is more effectively suppressed.

In the hook-and-loop fastener female member of the present invention, the ratio of the area of a welded portion formed by the embossed pattern to the area of the entire surface of the hook-and-loop fastener female member (hereinafter sometimes referred to as "embossing-welded area ratio") is from 12% to 23%. When the embossing-welded area ratio falls within the above-mentioned range, the hook-and-loop fastener female member of the present invention is a hook-and-loop fastener female member including an engaging layer adopting a non-woven fabric in which fluffing of the engaging layer after peeling after engagement with a hook-and-loop fastener male member can be more effectively suppressed.

In the hook-and-loop fastener female member of the present invention, the thickness of the welded portion formed by the embossed pattern is preferably 100 µm or less, more preferably from 10 µm to 100 µm, still more preferably from 15 µm to 80 µm, particularly preferably from 20 µm to 70 µm, most preferably from 25 µm to 60 µm. When the thickness of the welded portion formed by the embossed pattern falls within the above-mentioned range, the hook-and-loop fastener female member of the present invention is a hook-and-loop fastener female member including an engaging layer adopting a non-woven fabric in which fluffing of the engaging layer after peeling after engagement with a hook-and-loop fastener male member can be more effectively suppressed.

### <<Production Method for Hook-and-loop Fastener Female Member of the Present Invention>>

As one preferred production method for the hook-and-loop fastener female member of the present invention, raw non-woven fabrics are laminated and subjected to embossing treatment with a pattern roll at any appropriate treatment temperature (e.g., 160°C), any appropriate linear pressure (e.g., 160N/mm), and any appropriate treatment speed (e.g., 10 m/minute) to provide a desired hook-and-loop fastener female member.

### <<Application of Hook-and-loop Fastener Female Member of the Present Invention>>

The hook-and-loop fastener female member of the present invention can provide a hook-and-loop fastener by being combined with a hook-and-loop fastener male member configured to engage with the hook-and-loop fastener female member. That is, a hook-and-loop fastener of the present invention includes the hook-and-loop fastener female member of the present invention and a hook-and-loop fastener male member configured to engage with the hook-and-loop fastener female member. In addition, the hook-and-loop fastener female member of the present invention can be used in any appropriate article in which the effect of the present invention can be effectively utilized. A typical example of such article is a sanitary article. That is, a sanitary article of the present invention includes the hook-and-loop fastener female member of the present invention. Examples of such sanitary article include a diaper (in particular, a disposable diaper), a supporter, and a mask.

### Examples

The present invention is hereinafter specifically described by way of Examples. However, the present invention is by no means limited to these Examples. In Examples and the like, test and evaluation methods are as described below. In addition, "part (s) " means "part (s) by weight" and "%" means "wt%" unless otherwise stated.

### <Fluffing Evaluation>

An obtained hook-and-loop fastener female member was cut to a size of 5 cmx5 cm and allowed to stand still on a commercially available disposable diaper. Meanwhile, a 25 mmxl3 mm male member having a guide made of a polyethylene film having a thickness of 60 µm (commercially available mechanical hook member for a hook-and-loop fastener) was prepared, and was allowed to stand still on the female member so that its 25 mm sides were parallel to the flow direction of the engaging layer of the female member, followed by pressure bonding with a finger. After that, the guide was manually peeled and a fluffing property was visually checked. This operation was repeatedly performed 5 times on the same site of the hook-and-loop fastener.

Evaluation was performed in accordance with the following criteria.
○○○: The degree of fluffing after 5 times of repeated peeling is nearly zero.
○○: The degree of fluffing after 5 times of repeated peeling is small.
○: The degree of fluffing after 5 times of repeated peeling is large, but the degree of fluffing after 3 times of repeated peeling is small.
Δ: The degree of fluffing after 3 times of repeated peeling is large, but the degree of fluffing after 1 time of peeling is small.
×: The degree of fluffing after 1 time of peeling is large.

### <Measurement of Fiber Diameter>

Through the use of a digital microscope "VHX-1000" manufactured by Keyence Corporation, a non-woven fabric surface was photographed at a magnification of 500, and fiber diameters were measured for N=5 or more with the image analysis software of the digital microscope. The average value of the measured fiber diameters was defined as a fiber diameter.

### <Thickness of Non-woven Fabric>

Through the use of a digital microscope "VHX-1000" manufactured by Keyence Corporation, a cross-section of a non-woven fabric was photographed at a magnification of 100, and the thickness of the non-woven fabric was measured with the image analysis software of the digital microscope. In the measurement of the thickness, parallel lines drawn so as to have 10 points of intersection with fiber in an upper portion and a lower portion of the cross-section of the non-woven fabric, respectively, were defined as an upper side and a lower side, respectively, and a length between the upper side and the lower side was defined as the thickness of the non-woven fabric.

### [Examples 1 to 18 and Comparative Example 1]

Raw non-woven fabrics shown in Table 1 and Table 2 were laminated, and were subjected to embossing treatment with an embossed pattern roll at a treatment temperature of 200°C, a linear pressure of 160 N/mm, and a treatment speed of 20 m/minute to provide a hook-and-loop fastener female member.

The embossed pattern used was the embossed pattern of an arc shape illustrated in FIG. 1, and the embossment width and the maximum value of the distance between two adjacent embossments on a line in the MD direction (maximum embossment-to-embossment distance L) were as shown in Table 1 and Table 2.

### [Examples 19 to 22 and Comparative Example 2]

Raw non-woven fabrics shown in Table 3 were laminated, and were subjected to embossing treatment with an embossed pattern roll at a treatment temperature of 200°C, a linear pressure of 160 N/mm, and a treatment speed of 20 m/minute to provide a hook-and-loop fastener female member.

The embossed pattern used was the embossed pattern of a rhombus shape illustrated in FIG. 2 or the embossed pattern of a grid shape illustrated in FIG. 3, and the embossment width and the maximum value of the distance between adjacent embossments on a line in the MD direction (maximum embossment-to-embossment distance L) were as shown in Table 3.

**Table 1**

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| Engaging layer | Construction | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric |
| | Production method | Spunbond ed | Spunbond ed | Spunbond ed | Spunbond ed | Air-thro ugh | Air-thro ugh | Air-thro ugh | Air-thro ugh | Air-thro ugh | Air-thro ugh | Air-thro ugh |
| | Web forming | Spunlaid | Spunlaid | Spunlaid | Spunlaid | Airlaid | Airlaid | Airlaid | Airlaid | Airlaid | Airlaid | Airlaid |
| | Fiber composition | PP | PP | PP | PP | PE/PP | PE/PP | PE/PP | PE/PET | PE/PET | PE/PET | PE/PET |
| | Fiber diameter [µm] | 21.8 | 21.8 | 21.8 | 21.8 | 25.2 | 25.2 | 20.0 | 31.6 | 27.2 | 27.2 | 31.3 |
| | Basis weight [g/m²] | 18 | 18 | 18 | 18 | 30 | 30 | 18 | 30 | 40 | 40 | 20 |
| | Thickness of region free of embossed pattern [mm] | 0.19 | 0.19 | 0.19 | 0.19 | 0.96 | 0.96 | 0.83 | 1.12 | 1.17 | 1.17 | 2.19 |
| | Density [kg/m³] | 94 | 94 | 94 | 94 | 31 | 31 | 22 | 27 | 34 | 34 | 9.1 |
| Physical property layer | Construction | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric | Non-wove n fabric |
| | Fiber composition | PP | PP | PP | PP | PE/PP | PE/PP | PE/PP | PE/PP | PE/PP | PE/PP | PE/PP |
| | Fiber diameter [µm] | 19.6 | 19.6 | 19.6 | 19.6 | 19.8 | 19.8 | 19.8 | 19.8 | 19.8 | 19.8 | 19.8 |
| | Basis weight [g/m²] | 14 | 14 | 14 | 14 | 15 | 15 | 15 | 15 | 15 | 15 | 15 |
| | Thickness of region free of embossed pattern [mm] | 0.15 | 0.15 | 0.15 | 0.15 | 0.14 | 0.14 | 0.14 | 0.14 | 0.14 | 0.14 | 0.14 |
| | Density [kg/m³] | 93 | 93 | 93 | 93 | 107 | 107 | 107 | 107 | 107 | 107 | 107 |
| Embossed pattern | | Arc | Arc | Arc | Arc | Arc | Arc | Arc | Arc | Arc | Arc | Arc |
| Embossment width W [mm] | | 1 | 1 | 1 | 1 | 1 | 1 | 1 | 1 | 1 | 1 | 1 |
| Maximum embossment-to-embossment distance L [mm] | | 3 | 6 | 9 | 14 | 3 | 6 | 3 | 3 | 3 | 14 | 3 |
| L/W ratio | | 3 | 6 | 9 | 14 | 3 | 6 | 3 | 3 | 3 | 14 | 3 |
| Welded area ratio (%) | | 25 | 14 | 10 | 7 | 25 | 14 | 25 | 25 | 25 | 7 | 25 |
| Fluffing | | ○○○ | ○○○ | ○○ | Δ | ○○ | ○○ | ○○ | ○ | ○ | Δ | ○ |

**Table 2**

| | | Example 12 | Example 13 | Example 14 | Example 15 | Example 16 | Example 17 | Example 18 | Comparative Example 1 |
|---|---|---|---|---|---|---|---|---|---|
| Engaging layer | Construction | Non-woven fabric | Non-woven fabric | Non-woven fabric | Non-woven fabric | Non-woven fabric | Non-woven fabric | Non-woven fabric | Non-woven fabric |
| | Production method | Thermal bond | Thermal bond | Thermal bond | Thermal bond | Spunbonded | Air-throug h | Thermal bond | Spunlace |
| | Web forming | Carded | Carded | Carded | Carded | Spunlaid | Airlaid | Spunlaid | Carded |
| | Fiber composition | PP | PP | PP | PP | PE/PP | PE/PP | PE/PP | PP |
| | Fiber diameter [µm] | 14.5 | 14.5 | 14.5 | 14.5 | 21.8 | 20.0 | 14.5 | 16.7 |
| | Basis weight [g/m²] | 24 | 24 | 24 | 24 | 18 | 18 | 24 | 30 |
| | Thickness of region free of embossed pattern [mm] | 0.42 | 0.42 | 0.42 | 0.42 | 0.19 | 0.83 | 0.42 | 0.86 |
| | Density [kg/m³] | 57 | 57 | 57 | 57 | 94 | 22 | 57 | 35 |
| Physical property layer | Construction | Non-woven fabric | Non-woven fabric | Non-woven fabric | Non-woven fabric | Non-woven fabric | Non-woven fabric | Non-woven fabric | Non-woven fabric |
| | Fiber composition | PP | PP | PP | PP | PP | PE/PP | PP | PP |
| | Fiber diameter [µm] | 19.6 | 19.6 | 19.6 | 19.6 | 19.6 | 19.8 | 19.6 | 18.5 |
| | Basis weight [g/m²] | 14 | 14 | 14 | 14 | 14 | 15 | 14 | 15 |
| | Thickness of region free of embossed pattern [mm] | 0.15 | 0.15 | 0.15 | 0.15 | 0.15 | 0.14 | 0.15 | 0.15 |
| | Density [kg/m³] | 93 | 93 | 93 | 93 | 93 | 107 | 93 | 100 |
| Embossed pattern | | Arc | Arc | Arc | Arc | Arc | Arc | Arc | Arc |
| Embossment width W [mm] | | 1 | 1 | 1 | 1 | 0.6 | 0.6 | 0.6 | 1 |
| Maximum embossment-to-embossment distance L [mm] | | 3 | 6 | 9 | 14 | 3.4 | 3.4 | 3.4 | 3 |
| L/W ratio | | 3 | 6 | 9 | 14 | 5.7 | 5.7 | 5.7 | 3 |
| Welded area ratio (%) | | 25 | 14 | 22 | 19 | 15 | 15 | 15 | 25 |
| Fluffing | | ○○○ | ○○ | ○ | Δ | ○○○ | ○ | ○○○ | × |

**Table 3**

| | | Example 19 | Example 20 | Example 21 | Example 22 | Comparative Example 2 |
|---|---|---|---|---|---|---|
| Engaging layer | Construction | Non-woven fabric | Non-woven fabric | Non-woven fabric | Non-woven fabric | Non-woven fabric |
| | Production method | Spunbonde d | Spunbonde d | Air-throu gh | Thermal bond | Spunlace |
| | Web forming | Spunlaid | Spunlaid | Airlaid | Carded | Carded |
| | Fiber composition | PP | PP | PE/PP | PP | PP |
| | Fiber diameter [µm] | 21.8 | 21.8 | 20.0 | 14.5 | 16.7 |
| | Basis weight [g/m²] | 18 | 18 | 18 | 24 | 30 |
| | Thickness of region free of embossed pattern [mm] | 0.20 | 0.19 | 0.95 | 0.42 | 0.86 |
| | Density [kg/m³] | 90 | 94 | 19 | 57 | 35 |
| Physical property layer | Construction | Non-woven fabric | Non-woven fabric | Non-woven fabric | Non-woven fabric | Non-woven fabric |
| | Fiber composition | PP | PP | PE/PP | PP | PP |
| | Fiber diameter [ µm] | 19.6 | 19.6 | 19.8 | 19.6 | 18.5 |
| | Basis weight [g/m²] | 14 | 14 | 15 | 14 | 15 |
| | Thickness of region free of embossed pattern [mm] | 0.15 | 0.15 | 0.14 | 0.15 | 0.15 |
| | Density [kg/m³] | 93 | 93 | 107 | 93 | 100 |
| Embossed pattern | | Rhombus | Grid | Rhombus | Rhombus | Rhombus |
| Embossment width W [mm] | | 1 | 1 | 1 | 1 | 1 |
| Maximum embossment-to-embossment distance L [mm] | | 6 | 14 | 6 | 6 | 6 |
| L/W ratio | | 6 | 14 | 6 | 6 | 6 |
| Welded area ratio (%) | | 25.7 | 19 | 25.7 | 25.7 | 25.7 |
| Fluffing | | ○○○ | Δ | ○ | ○○○ | × |

### Industrial Applicability

The hook-and-loop fastener female member of the present invention can be used for any appropriate article in which the effect of the present invention can be effectively utilized. A typical example of such article is a sanitary article. Examples of such sanitary article include a diaper (in particular, a disposable diaper), a supporter, and a mask.

### Reference Signs List

- **100**: hook-and-loop fastener female member
- **10**: embossment
- **20**: region free of embossed pattern

## Claims

1. A hook-and-loop fastener female member (100), comprising:
an engaging layer engageable with a hook-and-loop fastener male member; and
a physical property layer configured to hold the engaging layer,
wherein the engaging layer includes a non-woven fabric of fiber,
wherein the hook-and-loop fastener female member (100) has an embossed pattern (10), wherein the embossed pattern (10) is an arc shape, and
wherein the hook-and-loop fastener female member (100) has a region free of the embossed pattern (20) in which pieces of the fiber have mutual bonding points,
wherein a distance L between two adjacent embossments (10) in a plurality of embossments (10) forming the embossed pattern (10) on any line in an MD direction is 10 mm or less,
wherein the diameter of the fiber of the non-woven fabric in the engaging layer is from 5 µm to 60 µm, and **characterized in that** the ratio of the area of the welded portion formed by the embossed pattern to the area of the entire surface of the hook-and-loop fastener female member is from 12% to 23%.

2. The hook-and-loop fastener female member (100) according to claim 1, wherein a distance L between two adjacent embossments (10) in a plurality of embossments (10) forming the embossed pattern (10) on any line in an MD direction is 7 mm or less.

3. The hook-and-loop fastener female member (100) according to claim 1, wherein a distance L between two adjacent embossments (10) in a plurality of embossments (10) forming the embossed pattern (10) on any line in an MD direction is 1 mm or more, preferably 3 mm or more.

4. The hook-and-loop fastener female member (100) according to claim 1, wherein an embossment width W of each of a plurality of embossments (10) forming the embossed pattern (10) is 0.1 mm or more, preferably 0.5 mm or more.

5. The hook-and-loop fastener female member (100) according to claim 1, wherein an embossment width W of each of a plurality of embossments (10) forming the embossed pattern (10) is 3.0 mm or less, preferably 1.5 mm or less.

6. The hook-and-loop fastener female member (100) according to claim 1, wherein a ratio L/W of a distance L between two adjacent embossments (10) in a plurality of embossments (10) forming the embossed pattern (10) to an embossment width W of each of the plurality of embossments (10) forming the embossed pattern (10) is from 0.3 to 100, preferably from 2 to 10.

7. The hook-and-loop fastener female member (100) according to claim 1, wherein a surface of the fiber and a surface of the physical property layer on an engaging layer side contain the same kind of polymer.

8. The hook-and-loop fastener female member (100) according to claim 7 wherein the polymer comprises polyolefin.

9. The hook-and-loop fastener female member (100) according to claim 1, wherein the non-woven fabric comprises a thermal point-bonded non-woven fabric, preferably a spunbonded non-woven fabric.

10. The hook-and-loop fastener female member (100) according to claim 1, wherein a total basis weight, which is a sum of a basis weight of the non-woven fabric in the engaging layer and a basis weight of a non-woven fabric in the physical property layer, is 60 g/m² or less, preferably 47 g/m² or less.

11. The hook-and-loop fastener female member (100) according to claim 1, wherein a total basis weight, which is a sum of a basis weight of the non-woven fabric in the engaging layer and a basis weight of a non-woven fabric in the physical property layer, is 30 g/m² or more.

12. A hook-and-loop fastener, comprising:
the hook-and-loop fastener female member (100) of claim 1; and
a hook-and-loop fastener male member configured to engage with the hook-and-loop fastener female member (100).

13. A sanitary article, comprising the hook-and-loop fastener female member (100) of claim 1.

## Patentansprüche

1. Klettverschluss-Schlaufenteil (100), umfassend:
eine Eingriffsschicht, die mit einem Klettverschluss-Hakenteil in Eingriff gebracht werden kann; und
eine physikalische Eigenschaftsschicht, die zum Halten der Eingriffsschicht konfiguriert ist,
wobei die Eingriffsschicht einen Faservliesstoff enthält,
wobei das Klettverschluss-Schlaufenteil (100) ein geprägtes Muster (10) aufweist, wobei das geprägte Muster (10) eine Bogenform ist, und
wobei das Klettverschluss-Schlaufenteil (100) einen Bereich frei von dem geprägten Muster (20) aufweist, in dem Stücke der Faser gegenseitige Bindungspunkte aufweisen,
wobei ein Abstand L zwischen zwei angrenzenden Prägungen (10) in einer Mehrzahl von Prägungen (10), welche das geprägte Muster (10) bilden, auf jeder Linie in einer MD-Richtung 10 mm oder weniger beträgt,
wobei der Durchmesser der Faser des Vliesstoffs in der Eingriffsschicht von 5 µm bis 60 µm beträgt, und
**dadurch gekennzeichnet, dass** das Verhältnis der Fläche des geschweißten Abschnitts, der durch das geprägte Muster gebildet ist, zu der Fläche der gesamten Oberfläche des Klettverschluss-Schlaufenteil von 12% bis 23% beträgt.

2. Klettverschluss-Schlaufenteil (100) nach Anspruch 1, wobei ein Abstand L zwischen zwei angrenzenden Prägungen (10) in einer Mehrzahl von Prägungen (10), die das geprägte Muster (10) bilden, auf jeder Linie in einer MD-Richtung 7 mm oder weniger beträgt.

3. Klettverschluss-Schlaufenteil (100) nach Anspruch 1, wobei ein Abstand L zwischen zwei angrenzenden Prägungen (10) in einer Mehrzahl von Prägungen (10), die das geprägte Muster (10) bilden, auf jeder Linie in einer MD-Richtung 1 mm oder mehr, vorzugsweise 3 mm oder mehr, beträgt.

4. Klettverschluss-Schlaufenteil (100) nach Anspruch 1, wobei eine Prägebreite W von jeder aus einer Mehrzahl von Prägungen (10), die das geprägte Muster (10) bilden, 0,1 mm oder mehr, vorzugsweise 0,5 mm oder mehr, beträgt.

5. Klettverschluss-Schlaufenteil (100) nach Anspruch 1, wobei eine Prägebreite W von jeder aus einer Mehrzahl von Prägungen (10), die das geprägte Muster (10) bilden, 3,0 mm oder weniger, vorzugsweise 1,5 mm oder weniger, beträgt.

6. Klettverschluss-Schlaufenteil (100) nach Anspruch 1, wobei ein Verhältnis L/W eines Abstands L zwischen zwei angrenzenden Prägungen (10) in einer Mehrzahl von Prägungen (10), die das geprägte Muster (10) bilden, zu einer Prägebreite W von jeder aus der Mehrzahl von Prägungen (10), die das geprägte Muster (10) bilden, von 0,3 bis 100, vorzugsweise von 2 bis 10, beträgt.

7. Klettverschluss-Schlaufenteil (100) nach Anspruch 1, wobei eine Oberfläche der Faser und eine Oberfläche der physikalischen Eigenschaftsschicht auf einer Eingriffsschichtseite die gleiche Art von Polymer enthalten.

8. Klettverschluss-Schlaufenteil (100) nach Anspruch 7, wobei das Polymer Polyolefin umfasst.

9. Klettverschluss-Schlaufenteil (100) nach Anspruch 1, wobei der Vliesstoff einen thermisch punktverfestigten Vliesstoff, vorzugsweise einen Spinnvliesstoff, umfasst.

10. Klettverschluss-Schlaufenteil (100) nach Anspruch 1, wobei ein Gesamtgrundgewicht, das eine Summe eines Grundgewichts des Vliesstoffs in der Eingriffsschicht und eines Grundgewichts eines Vliesstoffs in der physikalischen Eigenschaftsschicht ist, 60 g/m² oder weniger, vorzugsweise 47 g/m² oder weniger, beträgt.

11. Klettverschluss-Schlaufenteil (100) nach Anspruch 1, wobei ein Gesamtgrundgewicht, das eine Summe eines Grundgewichts des Vliesstoffs in der Eingriffsschicht und eines Grundgewichts eines Vliesstoffs in der physikalischen Eigenschaftsschicht ist, 30 g/m² oder mehr beträgt.

12. Klettverschluss, umfassend:
das Klettverschluss-Schlaufenteil (100) nach Anspruch 1; und
ein Klettverschluss-Hakenteil, das so konfiguriert ist, dass es mit dem Klettverschluss-Schlaufenteil (100) in Eingriff kommt.

13. Hygieneartikel, umfassend das Klettverschluss-Schlaufenteil (100) nach Anspruch 1.

## Revendications

1. Élément femelle d'une fixation par crochets et boucles (100), comprenant:
une couche prenante pouvant être prise à l'aide d'un élément mâle d'une fixation par crochets et boucles; et
une couche de propriété physique configurée pour maintenir la couche prenante,
dans lequel la couche prenante comprend un tissu non tissé de fibre,
dans lequel l'élément femelle d'une fixation par crochets et boucles (100) présente un motif bosselé (10), dans lequel le motif bosselé (10) a forme d'arc, et
dans lequel l'élément femelle d'une fixation par crochets et boucles (100) possède une zone exempte du motif bosselé (20) dans laquelle des morceaux de fibre présentent des points de liaison mutuelle,
dans lequel une distance L entre deux bosses adjacentes (10) dans une pluralité de bosses (10) formant le motif bosselé (10) sur une quelconque ligne dans la direction MD est 10 mm ou moins,
dans lequel le diamètre de la fibre du tissu non tissé dans la couche prenante va de 5 µm à 60 µm, et
**caractérisé en ce que** le rapport de la zone de la partie soudée formée par le motif bosselé à la zone de toute la surface de l'élément femelle d'une fixation par crochets et boucles, va de 12% à 23%.

2. Élément femelle d'une fixation par crochets et boucles (100) selon la revendication 1, dans lequel une distance L entre deux bosses adjacentes (10) dans une pluralité de bosses (10) formant le motif bosselé (10) sur une quelconque ligne dans la direction MD est 7 mm ou moins.

3. Élément femelle d'une fixation par crochets et boucles (100) selon la revendication 1, dans lequel une distance L entre deux bosses adjacentes (10) dans une pluralité de bosses (10) formant le motif bosselé (10) sur une quelconque ligne dans la direction MD est 1 mm ou plus, de préférence de 3 mm ou plus.

4. Élément femelle d'une fixation par crochets et boucles (100) selon la revendication 1, dans lequel une largeur de bosse W de chacune de la pluralité de bosses (10) formant le motif bosselé (10) est de 0,1 mm ou plus, de préférence de 0,5 mm ou plus.

5. Élément femelle d'une fixation par crochets et boucles (100) selon la revendication 1, dans lequel une largeur de bosse W de chacune de la pluralité de bosses (10) formant le motif bosselé (10) est de 3,0 mm ou moins, de préférence de 1,5 mm ou moins.

6. Élément femelle d'une fixation par crochets et boucles (100) selon la revendication 1, dans lequel un rapport L/W d'une distance L entre deux bosses adjacentes (10) dans la pluralité de bosses (10) formant le motif bosselé (10) à une largeur de bosse W de chacune de la pluralité des bosses (10) formant le motif bosselé (10), va de 0,3 à 100, de préférence de 2 à 10.

7. Élément femelle d'une fixation par crochets et boucles (100) selon la revendication 1, dans lequel une surface de la fibre et une surface de la couche de propriété physique sur un côté couche prenante contiennent le même type de polymère.

8. Élément femelle d'une fixation par crochets et boucles (100) selon la revendication 7 dans lequel le polymère comprend de la polyoléfine.

9. Élément femelle d'une fixation par crochets et boucles (100) selon la revendication 1, dans lequel le tissu non tissé comprend un tissu non tissé thermocollé par points, de préférence un tissu non tissé filé-lié.

10. Élément femelle d'une fixation par crochets et boucles (100) selon la revendication 1, dans lequel le poids de base total, lequel est la somme d'un poids de base du tissu non tissé dans la couche prenante et d'un poids de base d'un tissu non tissé dans la couche de propriété physique, est de 60 g/m² ou moins, de préférence de 47 g/m² ou moins.

11. Élément femelle d'une fixation par crochets et boucles (100) selon la revendication 1, dans lequel le poids de base total, lequel est la somme d'un poids de base du tissu non tissé dans la couche prenante et d'un poids de base d'un tissu non tissé dans la couche de propriété physique, est de 30 g/m² ou plus.

12. Fixation par crochets et boucles, comprenant:
l'élément femelle d'une fixation par crochets et boucles (100) selon la revendication 1; et
un élément mâle d'une fixation par crochets et boucles configuré pour se prendre avec l'élément femelle d'une fixation par crochets et boucles (100).

13. Article sanitaire, comprenant l'élément femelle d'une fixation par crochets et boucles (100) selon la revendication 1.
